# EUROPEAN PATENT APPLICATION

(11) **EP 4 722 348 A1**
(43) Date of publication of application: **08.04.2026**
(21) Application number: 24815621.8
(22) Date of filing: 31.05.2024
(51) Int. Cl.: C12N 5/0783, A61K 35/17, A61P 35/00, C12N 5/10

(54) **T CELL PROGENITOR PRODUCTION METHOD**

(30) Priority: 02.06.2023 JP 2023091294
(71) Applicant: Kyoto University, Kyoto-shi, Kyoto 606-8501 (JP); Takeda Pharmaceutical Company Limited, Osaka-shi, Osaka 541-0045 (JP)
(72) Inventor: KANEKO, Shin, Kyoto-shi, Kyoto 606-8501 (JP); IRIGUCHI, Shoichi, Kyoto-shi, Kyoto 606-8501 (JP)
(74) Representative: Henderson, Helen Lee
(86) International application number: PCT/JP2024/020073
(87) International publication number: WO 2024/248141

(57) **Abstract**

Disclosed are a method for producing a cell population containing a T cell progenitor, the method comprising the following step: (1) culturing a cell population containing a CD34⁺ cell in the presence of an aryl hydrocarbon receptor antagonist and an LSD1 degradation inducer, as well as a cell population containing a T cell progenitor obtained by the method, a medicine comprising the cell population containing a T cell progenitor, and a T cell progenitor inducer comprising an aryl hydrocarbon receptor antagonist and an LSD1 degradation inducer.

## Description

### Technical Field

The present invention relates to a method for producing a cell population containing a T cell progenitor, a cell population containing a T cell progenitor obtained by the method, a medicine comprising the cell population containing a T cell progenitor, a T cell progenitor inducer, and the like.

### Background Art

Hematopoietic stem cell transplantation (HSCT) is a current standard treatment for many malignant and non-malignant hematopoietic diseases. Before infusion (transplant) of hematopoietic stem cells (HSC) (donor graft), the patient must undergo a pretreatment regimen, including chemotherapy or chemoradiotherapy. This pretreatment causes serious damage to the thymic microenvironment and thus delays the rearrangement of peripheral CD4⁺ and CD8⁺ T cells, making the patient vulnerable to opportunistic infections. The component of the thymic microenvironment most vulnerable to chemoradiotherapy is thymic epithelial cells, and the damage in thymic epithelial cells causes long-term impairment in thymus formation after transplantation.

For the problems with thymocyte development caused by the pretreatment, the therapy of infusing in vitro-derived T cell progenitors (proT) by transplanting hematopoietic stem cells is known to promote the recovery of the thymus damaged by exposure to irradiation. Aiming at recovering T cells damaged by thymic disorders after radiotherapy, the research and development of cell therapies using T cell progenitors (proT therapy) has been underway.

NPL 1 discloses that the co-addition of SR1 and UM171 can stimulate the in vitro proliferation of hematopoietic stem cells derived from human umbilical cord blood.

PTL 1 discloses UM171 as a compound that promotes expansion of CD34⁺ cells.

The practical application of proT therapies in the future will demand industrial methods for producing proT with high production efficiency (yield). However, there have been no reports on such production methods.

### Citation List

### Patent Literature

PTL 1: WO2019/067811

### Non-patent Literature

NPL 1: Fares et al., Science, 345 (6203), 1509-1512, 2014

### Summary of Invention

### Technical Problem

An object of the present invention is to provide a method for producing a T cell progenitor that enables the production of a cell population containing a T cell progenitor with high efficiency, a T cell progenitor inducer, and the like.

### Solution to Problem

The present inventors conducted extensive research to achieve the object and found that T cell progenitor differentiation can be achieved with high efficiency by culturing CD34⁺ cells (in particular, CD34⁺/CD43⁻/CD184⁻/CD73⁻ cells) in the presence of an aryl hydrocarbon receptor antagonist (e.g., SR1) and an LSD1 degradation inducer (e.g., UM171).

The present invention was completed by further research based on these findings and provides the following method for producing a T cell progenitor, T cell progenitor, medicine, T cell progenitor inducer, and the like.

[1] A method for producing a cell population containing a T cell progenitor, the method comprising the following step: (1) culturing a cell population containing a CD34⁺ cell in the presence of an aryl hydrocarbon receptor antagonist and an LSD1 degradation inducer.
[2] The method according to [1], wherein the aryl hydrocarbon receptor antagonist is Stemregenin-1 (SR1).
[3] The method according to [1] or [2], wherein the LSD1 degradation inducer is UM171.
[4] The method according to any one of [1] to [3], wherein step (1) is a step of culturing a CD34⁺ cell in the further presence of fibronectin or a fragment of fibronectin, and/or Delta-like 4 (DLL4).
[5] The method according to [4], wherein step (1) is a step of culturing a cell population containing a CD34⁺ cell in the further presence of at least one member selected from the group consisting of a stem cell factor (SCF), interleukin-3 (IL-3), and interleukin-6 (IL-6).
[5a] The method according to any one of [1] to [5], wherein the medium used in step (1) contains IMDM, L-alanyl-L-glutamine (and/or GlutaMAX (trade name)), and albumin.
[6] The method according to any one of [1] to [5a], wherein the CD34⁺ cell is a CD34⁺/CD43⁻/CD184⁻/CD73⁻ cell.
[7] The method according to any one of [1] to [6], wherein the cell population containing a CD34⁺ cell is derived from an induced pluripotent stem cell (an iPS cell).
[8] The method according to any one of [1] to [7], wherein the T cell progenitor comprises a foreign gene encoding a receptor (particularly a chimeric antigen receptor).
[8a] The method according to any one of [1] to [8], further comprising step (A) of preparing an iPS cell, wherein in step (1), a cell population containing a CD34⁺ cell derived from the iPS cell is used.
[8b] The method according to any one of [1] to [8a], further comprising step (B) of inducing differentiation of an iPS cell into a cell population containing a CD34⁺ cell, wherein in step (1), the cell population containing a CD34⁺ cell is used.
[8c] The method according to [8b], further comprising step (C) of selecting a cell population containing a hemogenic endothelial cell (a CD34⁺/CD43⁻/CD184⁻/CD73⁻ cell) from the cell population containing a CD34⁺ cell obtained in step (B).
[9] A cell population containing a T cell progenitor obtained by the method according to any one of [1] to [8c].
[10] A medicine comprising the cell population containing a T cell progenitor of [9].
[11] The medicine according to [10], which is a preventive and/or therapeutic agent for cancer.
[12] A T cell progenitor inducer comprising an aryl hydrocarbon receptor antagonist and an LSD1 degradation inducer.
[13] The inducer according to [12], wherein the aryl hydrocarbon receptor antagonist is SR1.
[14] The inducer according to [12] or [13], wherein the LSD1 degradation inducer is UM171.
[15] The inducer according to any one of [12] to [14], further comprising fibronectin or a fragment of fibronectin, and/or DLL4.
[16] The inducer according to [15], further comprising at least one member selected from the group consisting of SCF, IL-3, and IL-6.
[17] A method for preventing and/or treating cancer, comprising administering the cell population containing a T cell progenitor of [9] to a subject in need thereof.
[18] The cell population containing a T cell progenitor of [9], for use in the prevention and/or treatment of cancer.
[19] Use of the cell population containing a T cell progenitor of [9] in the production of a medicine for preventing and/or treating cancer.
[20] A medium for T cell progenitor differentiation, comprising an aryl hydrocarbon receptor antagonist and an LSD1 degradation inducer.
[21] The medium according to [20], wherein the aryl hydrocarbon receptor antagonist is SR1.
[22] The medium according to [20] or [21], wherein the LSD1 degradation inducer is UM171.
[23] The medium according to any one of [20] to [22], further comprising fibronectin or a fragment of fibronectin, and/or DLL4.
[24] The medium according to any one of [20] to [23], further comprising at least one member selected from the group consisting of SCF, IL-3, and IL-6.
[25] A kit for T cell progenitor differentiation, comprising an aryl hydrocarbon receptor antagonist and an LSD1 degradation inducer.
[26] The kit according to [25], wherein the aryl hydrocarbon receptor antagonist is SR1.
[27] The kit according to [25] or [26], wherein the LSD1 degradation inducer is UM171.
[28] The kit according to any one of [25] to [27], further comprising fibronectin or a fragment of fibronectin, and/or DLL4.
[29] The kit according to any one of [25] to [28], further comprising at least one member selected from the group consisting of SCF, IL-3, and IL-6.

### Advantageous Effects of Invention

The present invention enables the production of a cell population containing T cell progenitors from a cell population containing CD34⁺ cells with high efficiency.

### Brief Description of Drawings

Fig. 1 shows the results of flow cytometry on an immunostained cell population containing HECs differentiated from induced pluripotent stem cells.
Fig. 2 shows the results of flow cytometry on an immunostained cell population containing T cell progenitors differentiated from induced pluripotent stem cells.
Fig. 3-1 shows the results of flow cytometry on an immunostained cell population containing T cell progenitors differentiated from induced pluripotent stem cells.
Fig. 3-2 shows graphs of the percentage of T cell progenitors in a cell population containing T cell progenitors differentiated from induced pluripotent stem cells. In the graphs, U stands for UM171 and S stands for SR1. Values in the graphs show mean ± SEM, and n=3.
Fig. 4-1 shows the results of flow cytometry on immunostained blood cells collected 8 weeks after transplanting a cell population containing T cell progenitors differentiated from induced pluripotent stem cells into mice.
Fig. 4-2 shows the results of flow cytometry on immunostained blood cells collected 12 weeks after transplanting a cell population containing T cell progenitors differentiated from induced pluripotent stem cells into mice.
Fig. 5 shows the results of flow cytometry on immunostained thymocytes collected 4 weeks after transplanting a cell population containing T cell progenitors differentiated from induced pluripotent stem cells into mice.
Fig. 6 shows the results of producing CAR-expressing T cell progenitors. Top: outline of the method for producing CAR-expressing T cell progenitors, Middle: results of flow cytometry on an immunostained cell population containing gene-transduced T cell progenitors, Bottom: graphs showing the number of lentivirus and CAR copies per cell.
Fig. 7 shows the results of administering a B cell tumor line to mice transplanted with CAR-expressing T cell progenitors. Top: outline of an experiment to assess the in vivo anticancer effect of CAR-expressing T cell progenitors, Bottom: results of flow cytometry on immunostained blood cells collected 8 to 12 weeks after transplanting a cell population containing CAR-expressing T cell progenitors into mice.
Fig. 8 shows the results of administering a B cell tumor line to mice transplanted with CAR-expressing T cell progenitors. Top: outline of an experiment to assess the in vivo anticancer effect of CAR-expressing T cell progenitors, Bottom: images of luciferase luminescence from B cell tumor line NALM-6 9 to 12 weeks after transplanting a cell population containing CAR-expressing T cell progenitors into mice.

### Description of Embodiments

Embodiments of the present invention are described below in detail.

The term "comprise(s)" or "comprising" means inclusion of the element(s) following the word without limitations thereto. Accordingly, this suggests inclusion of the element(s) following the word, but does not suggest exclusion of any other element. The phrase "consist(s) of" or "consisting of" means inclusion of all the element(s) following the phrase and limitation thereto. Accordingly, the phrase "consist(s) of" or "consisting of" indicates that the enumerated element(s) are required or essential and that substantially no other elements exist. The phrase "consist(s) essentially of" or "consisting essentially of" means inclusion of any element following the phrase and limitation of other elements to those that do not affect the activity or effect of the enumerated element(s) specified in the present disclosure. Accordingly, the phrase "consist(s) essentially of" or "consisting essentially of" indicates that the enumerated element(s) are required or essential, but other elements are optional and may exist or not exist depending on whether they affect the activity or effect of the enumerated element(s).

In the present specification, the term "culture" refers to maintenance or/and proliferation of cells in an in vitro environment. The term "culturing" refers to maintaining or/and proliferating cells outside tissue or outside the body (e.g., in a cell culture dish or flask).

In the present specification, the phrase "culturing cells in the presence of a substance" refers to, for example, culturing cells in a medium that contains the substance. Such culture may be, for example, culture in a medium containing only the substance, or in the presence of the substance together with other differentiation-inducing factors. When adding the substance to a medium, it can be added directly to the medium, or dissolved in an appropriate solvent immediately before use and then added to the medium. The substance can also be immobilized on the surface of a substrate or carrier during culture.

In the present specification, the term "positive (+)" means that a protein or gene is expressed in levels detectable by methods known in the art. In the case of a protein that is expressed intracellularly and does not appear on the cell surface (e.g., a transcription factor or a subunit thereof), a reporter protein is expressed together with the protein, and the reporter protein is detected, whereby the target protein can be detected. Gene detection can be performed by using nucleic acid amplification and/or nucleic acid detection methods, such as RT-PCR, microarray, biochip, and RNA-Seq.

In the present specification, the term "negative (-)" means that the expression level of a protein or gene is below the lower limit of detection by all or any of the known methods as described above, or that its expression level is low. The lower limit of detection of protein or gene expression may vary depending on the method. The level of protein or gene expression (whether it is low or high expression) can be determined by comparing the results with those of control cells measured under the same conditions. For example, the level of CD7 expression in a cell population can be determined by comparing the amount of CD7 expression in the cell population with that of a control using flow cytometry. If the expression is equivalent to that of the control, it is considered to be low expression, and if the expression is higher than that of the control cells, it is considered to be high expression.

In the present specification, the term "marker" refers to a protein or its gene that is specifically expressed on the cell surface, in the cytoplasm, in the nucleus, or the like in a given cell type. The marker is preferably a "cell surface marker." The term "cell surface marker" refers to a protein expressed on the cell surface that can be labeled (stained) with fluorescent substances and that facilitates the detection, condensation, isolation, or the like of cells expressing a cell surface marker. The cell surface marker refers to a gene that is expressed (positive marker) or not expressed (negative marker) specifically in a given cell type, and specifically a substance that is produced (positive marker) or not produced (negative marker) as mRNA by transcription of the gene in a genome or as a protein by translation of the mRNA.

Such cell surface markers can be detected by immunological assays using antibodies specific for the cell surface markers, such as ELISA, immunostaining, and flow cytometry.

In the present specification, the term "expression" is defined as the transcription and/or translation of a specific nucleotide sequence driven by an intracellular promoter.

In the present specification, the term "pluripotent stem cells" refers to embryonic stem cells (ES cells) and cells with pluripotency similar to that of ES cells, i.e., cells that have the potential to differentiate into various biological tissues (including all of endoderm, mesoderm, and ectoderm). Examples of cells with pluripotency similar to that of ES cells include induced pluripotent stem cells (which are sometimes referred to as "iPS cells" in this specification).

In the present specification, the term "hematopoietic stem cells" refers to multipotent stem cells that can be differentiated into blood cells, and are CD45-positive and CD34-positive cells. Hematopoietic stem cells are present mainly in the bone marrow in the human body and differentiated into leucocytes (neutrophils, eosinophils, basophils, lymphocytes, monocytes, and macrophages), erythrocytes, platelets, mast cells, dendritic cells, etc. In the present invention, hematopoietic stem cells may be cells isolated from a biological tissue such as bone marrow, or cells derived from ES cells or iPS cells.

In the present specification, the term "hematopoietic progenitor cells" refers to cells that can be differentiated into cells such as lymphocytes, eosinophils, neutrophils, basophils, erythrocytes, or megakaryocytes. Hematopoietic progenitor cells can be recognized by their surface antigen CD34 and/or CD43 being positive.

In the present specification, "T cell progenitors" (which are sometimes referred to as "proT cells" or "proT" in the present specification) refer to hematopoietic cells produced in the process of differentiation from hematopoietic stem cells (HSCs) into CD3-positive T cells in a human living body, and are CD34-positive and CD7-positive (CD34⁺/CD7⁺ cells), and further can be CD144-positive (CD34⁺/CD7⁺/CD144⁺ cells). Further, in the present specification, proT can be CD43-negative, CD1a-negative, and/or CD116-negative.

In the present specification, the term "cell population" refers to two or more cells of the same or different kind. The term "cell population" also refers to a mass of cells of the same or different kind.

The CD34⁺ cells for use in the present invention are not particularly limited and may be, for example, those induced in vitro according to known methods (e.g., CD34-positive hemogenic endothelial cells (HECs) induced from pluripotent stem cells), or CD34⁺ cells isolated from biological tissue by known methods.

CD34⁺ cells can be produced by subjecting pluripotent stem cells to a known method. If the pluripotent stem cells are iPS cells, CD34⁺ cells can be produced, for example, by inducing differentiation of hematopoietic progenitor cells according to the methods disclosed in WO2017/221975, WO2018/135646, or Cell Reports 2 (2012) 1722-1735.

The biological tissue from which CD34⁺ cells are isolated can be any tissue containing CD34⁺ cells, and can be, for example, bone marrow, umbilical cord blood, blood, or thymus.

The various cells for use in the present invention are preferably cells certified by the Good Manufacturing Practice (GMP) standards from the standpoint of therapeutic applications.

Examples of pluripotent stem cells include embryonic stem cells (ES cells), induced pluripotent stem cells (iPS cells), embryonic carcinoma cells (EC cells), and embryonic germ stem cells (EG cells). The pluripotent stem cells are preferably iPS cells (more preferably human iPS cells). If the pluripotent stem cells are ES cells or any cells derived from a human embryo, those cells may be produced by destroying the embryo or without destroying the embryo, and are preferably produced without destroying the embryo.

For "ES cells," various mouse ES cell lines established by inGenious Targeting Laboratory, RIKEN (Institute of Physical and Chemical Research), and the like are available as mouse ES cells; and various human ES cell lines established by the University of Wisconsin, NIH, RIKEN, Kyoto University, the National Center for Child Health and Development, Cellartis, and the like are available as human ES cells. For example, human ES cell lines for use may be CHB-1 to CHB-12, RUES1, RUES2, HUES1 to HUES28, and other cell lines distributed by ESI Bio; H1, H9, and other cell lines distributed by WiCell Research Institute; and KhES-1, KhES-2, KhES-3, KhES-4, KhES-5, SSES1, SSES2, SSES3, and other cell lines distributed by RIKEN.

The term "induced pluripotent stem cells" refers to cells that are obtained by reprogramming mammalian somatic cells or undifferentiated stem cells by introducing specific factors (nuclear reprogramming factors). Various induced pluripotent stem cells are available at present. Usable induced pluripotent stem cells include iPS cells established by Yamanaka et al. by introducing four factors, Oct3/4, Sox2, Klf4, and c-Myc, into mouse fibroblasts (Takahashi K, Yamanaka S., Cell, (2006) 126: 663-676); iPS cells derived from human cells, established by introducing similar four factors into human fibroblasts (Takahashi K, Yamanaka S., et al. Cell, (2007) 131: 861-872); Nanog-iPS cells established by sorting cells using expression of Nanog as an indicator after introduction of the 4 factors (Okita, K., Ichisaka, T., and Yamanaka, S. (2007), Nature 448, 313-317); iPS cells produced by a method not using c-Myc (Nakagawa M, Yamanaka S., et al., Nature Biotechnology, (2008) 26, 101-106); and iPS cells established by introducing 6 factors by a virus-free method (Okita K et al., Nat. Methods 2011 May; 8 (5): 409-12, Okita K et al. Stem Cells. 31 (3): 458-66). It is also possible to use induced pluripotent stem cells established by Thomson et al. by introducing 4 factors OCT3/4, SOX2, NANOG, and LIN28 (Yu J., Thomson JA. et al., Science (2007) 318: 1917-1920); induced pluripotent stem cells produced by Daley et al. (Park IH, Daley GQ. et al., Nature (2007) 451: 141-146); induced pluripotent stem cells produced by Sakurada et al. (JP2008-307007A); and the like.

Additionally, usable induced pluripotent stem cells also include any induced pluripotent stem cells known in the art and disclosed in all published articles (e.g., Shi Y., Ding S., et al., Cell Stem Cell, (2008) Vol. 3, Issue 5, 568-574; Kim JB., Scholer HR., et al., Nature, (2008) 454, 646-650; Huangfu D., Melton, DA., et al., Nature Biotechnology, (2008) 26, No. 7, 795-797), as well as patent documents (e.g., JP2008-307007A, JP2008-283972A, US Patent Publication No. 2008/2336610, US Patent Publication No. 2009/047263, WO2007/069666, WO2008/118220, WO2008/124133, WO2008/151058, WO2009/006930, WO2009/006997, and WO 2009/007852).

Available induced pluripotent stem cell lines include various iPS cell lines established by NIH, RIKEN, Kyoto University, and the like. Examples of human iPS cell lines include HiPS-RIKEN-1A, HiPS-RIKEN-2A, HiPS-RIKEN-12A, and Nips-B2 from RIKEN; and Ff-I01s04, Ff-WJc513, QHJI, RWMH, DRXT, RJWI, YZWJ, ILCL, GLKV, 253G1, 201B7, 409B2, 454E2, 606A1, 610B1, and 648A1 from Kyoto University.

The "nucleic acid" may be any molecule obtained by polymerizing a nucleotide and a molecule having the same function as the nucleotide. Examples include RNA that is a polymer of ribonucleotide, DNA that is a polymer of deoxyribonucleotide, a polymer that is a mixture of ribonucleotide and deoxyribonucleotide, and a nucleotide polymer containing a nucleotide analog, and the nucleic acid may also be a nucleotide polymer containing a nucleic acid derivative. The nucleic acid may be a single-stranded nucleic acid or a double-stranded nucleic acid. Double-stranded nucleic acids include a double-stranded nucleic acid in which one strand hybridizes to the other strand under stringent conditions.

The nucleotide analog may be any molecule as long as it is a molecule obtained by modifying ribonucleotide, deoxyribonucleotide, RNA, or DNA, for improvement of nuclease resistance, stabilization, increase in affinity with complementary strand nucleic acids, enhancement of cell permeability, or visualization, compared with RNA or DNA. The nucleotide analog may be a naturally occurring molecule or a non-natural molecule. Examples include sugar-modified nucleotide analogs (e.g., 2'-O-methylribose-substituted nucleotide analog, 2'-O-propylribose-substituted nucleotide analog, 2'-methoxyethoxyribose-substituted nucleotide analog, 2'-O-methoxyethylribose-substituted nucleotide analog, 2'-O-[2-(guanidium)ethyl]ribose-substituted nucleotide analog, 2'-fluororibose-substituted nucleotide analog, bridged nucleic acid (BNA), locked nucleic acid (LNA), ethylene-bridged nucleic acid (ENA), peptide nucleic acid (PNA), oxy-peptide nucleic acid (OPNA), and peptide ribonucleic acid (PRNA)), phosphodiester bond-modified nucleotide analogs (e.g., phosphorothioate bond-substituted nucleotide analog and N3'-P5' phosphoramidate bond-substituted nucleotide analog), and the like.

The nucleic acid derivative may be any molecule as long as it is a molecule obtained by adding another chemical substance to a nucleic acid, for improvement of nuclease resistance, stabilization, increase in affinity with complementary strand nucleic acids, enhancement of cell permeability, or visualization, compared with the nucleic acid, and specific examples include 5'-polyamine-adduct derivatives, cholesterol-adduct derivatives, steroid-adduct derivatives, bile acid-adduct derivatives, vitamin-adduct derivatives, Cy5-adduct derivatives, Cy3-adduct derivatives, 6-FAM-adduct derivatives, biotin-adduct derivatives, and the like.

The method for producing a cell population containing T cell progenitors according to the present invention (which is sometimes referred to simply as "the production method of the present invention" in the present specification) includes the following step:
(1) culturing a cell population containing CD34⁺ cells in the presence of an aryl hydrocarbon receptor antagonist and an LSD1 degradation inducer

As the medium for culturing the cell population containing CD34⁺ cells, a medium used in culturing animal cells can be used as a basal medium. The basal medium can be any medium that can be used in culturing animal cells. Examples of basal mediums include Iscove's Modified Dulbecco's Medium (IMDM), Medium 199, Eagle's Minimum Essential Medium (EMEM), αMEM, Dulbecco's Modified Eagle's Medium (DMEM), Ham's F12, RPMI 1640, Fischer's medium, MCDB 131, Neurobasal Medium (Life Technologies), StemSpan SFEM II (STEMCELL Technologies), and mixed mediums of these. The medium for culturing the cell population containing CD34⁺ cells is particularly a medium containing IMDM, L-alanyl-L-glutamine (e.g., GlutaMAX (trade name)), and albumin (e.g., human albumin).

The aryl hydrocarbon receptor (AHR) is a transcription factor that belongs to the Per-Arnt-Sim (PAS) family. The AHR is inactive when not bound to a ligand. When an aromatic hydrocarbon compound binds to the AHR as a ligand, the AHR translocates into the nucleus and forms a heterodimer with a molecule called "AhR nuclear translocator" (ARNT) in the nucleus. The heterodimer binds to a xenobiotic response element (XRE) on DNA to activate transcription. The aryl hydrocarbon receptor antagonist (AHR antagonist) refers to a substance that inhibits at least partly or entirely the reaction that occurs when an agonist binds to AHR. The AHR antagonist may be one that acts directly on AHR, or one that acts indirectly on AHR through action on another substance.

The aryl hydrocarbon receptor antagonist is not particularly limited and includes low-molecular-weight compounds, high-molecular-weight compounds, proteins such as antibodies and fragments of antibodies, peptides, and nucleic acids. Specifically, the aryl hydrocarbon receptor antagonist include SR1 (4-(2-((2-(benzo[b]thiophen-3-yl)-9-isopropyl-9H-purin-6-yl)amino)ethyl)phenol), α-naphthoflavone, 1,4-dihydroxyanthraquinone, 1,5-dihydroxyanthraquinone, 1,8-dihydroxyanthraquinone, galangin, resveratrol, PD98059, CH-223191, GNF-351, TSU-16, 6,2',4'-trimethoxyflavone, 3',4'-dimethoxyflavone, and compounds disclosed as AHR antagonists in WO2012/015914. In particular, the aryl hydrocarbon receptor antagonist is preferably SR1.

The AHR antagonist in a medium can be of any concentration as long as T cell progenitors can be produced, and the concentration can be appropriately adjusted according to the type of the AHR antagonist for use. For example, if the AHR antagonist is SR1, the concentration of the AHR antagonist can be 0.1 nM to 3000 nM, preferably 10 nM to 2000 nM, and more preferably 100 nM to 1500 nM.

LSD1 (Lysine-specific demethylase 1) is an enzyme that demethylates mono- or dimethylated lysine residues of histones and is also a component protein of various corepressor complexes. This property allows it to epigenetically regulate genes. The LSD1 degradation inducer refers to a substance that induces the degradation of LSD1. The LSD1 degradation inducer may also be referred to as a CoREST degradation inducer. The LSD1 degradation inducer may act directly on LSD1, or may indirectly induce the degradation of LSD1 through action on another substance.

The LSD1 degradation inducer is not particularly limited, and examples include low-molecular-weight compounds, high-molecular-weight compounds, proteins such as antibodies and fragments thereof, peptides, and nucleic acids. Specific examples include UM171 (4-N-[2-benzyl-7-(2-methyltetrazol-5-yl)-9H-pyrimido[4,5-b]indol-4-yl]cyclohexane-1,4-diamine) and UM729 (methyl 4-((3-(piperidin-1-yl)propyl)amino)-9H-pyrimido[4,5-b]indole-7-carboxylate). Preferred among these is UM171.

The concentration of the LSD1 degradation inducer in a medium is not particularly limited as long as T cell progenitors can be produced, and the concentration can be appropriately adjusted according to the type of the LSD1 degradation inducer for use. For example, if the LSD1 degradation inducer is UM171, the concentration of the LSD1 degradation inducer can be 1.0 nM to 300 nM, and preferably 3.0 nM to 100 nM.

In step (1), the cell population containing CD34⁺ cells can be cultured in the further presence of fibronectin or a fragment of fibronectin, and/or DLL4. In step (1), it is preferable to culture the cell population containing CD34⁺ cells in the further presence of two or more of (i) fibronectin or a fragment of fibronectin, and (ii) DLL4.

The fragment of fibronectin (FN) can be selected from the fragments contained in the FN-binding domain, cell adhesion domain, or heparin-binding domain. The fragment of fibronectin can be a fragment containing, for example, at least one fragment selected from III₁, III₂, III₃, III₇, III₈, III₉, III₁₁, III₁₂, III₁₃, and CS-1, and can also be a fragment of repeatedly linked multiple domains. The fragment of fibronectin for use can be, for example, a fragment containing a cell adhesion domain containing a ligand for VLA-5, a heparin-binding domain, CS-1 domain, which is a ligand for VLA-4, or III₁ etc. Additionally, examples of the fragment of fibronectin also include CH-271, CH-296, H-271, and H-296 disclosed in J. Biochem., Vol. 110, pp. 284-291 (1991), derivatives of these, and modified products of these. CH-296 is commercially available under the trade name of RetroNectin. The two-thirds of the polypeptide from the C-terminus of III₁ is also commercially available under the name of Fibronectin Fragment III₁-C.

FN and a fragment of FN for use may be immobilized on an appropriate solid phase, such as a cell culture vessel material or a cell culture carrier (e.g., beads, a membrane, or a glass slide). FN and/or a fragment of FN can be immobilized on a solid phase in accordance with the method disclosed in, for example, WO00/09168A. FN and/or a fragment of FN in a medium can be of any concentration as long as T cell progenitors can be produced. For example, the concentration of FN and/or a fragment of FN is preferably 0.001 µg/mL to 500 µg/mL, and more preferably 0.01 µg/mL to 500 µg/mL. If FN and a fragment of FN is immobilized for use, immobilization on a solid phase can be performed by using a solution of FN and a fragment of FN in the concentration described above. Culture of a cell population in the presence of FN and a fragment of FN is disclosed in WO03/080817A, and can be performed with reference to this document. DLL4 for use can also be immobilized on a cell culture carrier in the same manner. If FN and a fragment of FN, and DLL4 are immobilized on a solid phase and used, they do not have to be contained in a medium for T cell progenitor differentiation. DLL4 in a medium can be of any concentration as long as T cell progenitors can be produced. For example, the concentration of DLL4 is preferably 0.001 µg/mL to 500 µg/mL, and more preferably 0.01 µg/mL to 500 µg/mL.

In step (1), CD34⁺ cells can be cultured in the further presence of a stem cell factor (SCF), IL-3, and IL-6. In step (1), two or more of (iii) SCF, (iv) IL-3, and (v) IL-6 are preferably contained, and three of them are more preferably contained.

The concentration of SCF in a medium can be, for example, 3 ng/mL to 3000 ng/mL, preferably 10 ng/mL to 1000 ng/mL, and more preferably 30 ng/mL to 300 ng/mL.

The concentration of IL-3 in a medium can be, for example, 0.5 ng/mL to 1000 ng/mL, preferably 1 ng/mL to 500 ng/mL, and more preferably 1 ng/mL to 300 ng/mL.

The concentration of IL-6 in a medium can be, for example, 0.5 ng/mL to 1000 ng/mL, preferably 1 ng/mL to 500 ng/mL, and more preferably 1 ng/mL to 300 ng/mL.

In step (1), the cell population containing CD34⁺ cells may be cultured in the presence of, for example, thrombopoietin (TPO), Flt3L (Fms-like tyrosine kinase 3 ligand), IL-7, and the like in addition to the ingredients described above.

The aryl hydrocarbon receptor antagonist, LSD1 degradation inducer, and the like can be used in free or salt form. Examples of salts include salts with inorganic bases, such as sodium salts, magnesium salts, potassium salts, calcium salts, and aluminum salts; salts with organic bases, such as methylamine salts, ethylamine salts, and ethanolamine salts; salts with basic amino acids, such as lysine, ornithine, and arginine; and ammonium salts. The salts may be acid addition salts, and specific examples of such salts include acid addition salts with mineral acids, such as hydrochloric acid, hydrobromic acid, hydroiodic acid, sulfuric acid, nitric acid, and phosphoric acid; with organic acids, such as formic acid, acetic acid, propionic acid, oxalic acid, malonic acid, malic acid, tartaric acid, fumaric acid, succinic acid, lactic acid, maleic acid, citric acid, methanesulfonic acid, and ethanesulfonic acid; and with acidic amino acids, such as aspartic acid and glutamic acid. The aryl hydrocarbon receptor antagonist, LSD1 degradation inducer, and the like also include hydrates, solvates, polymorphs, etc.

The medium for culturing the cell population containing CD34⁺ cells may contain serum or may be serum-free, and is preferably serum-free.

The medium for culturing the cell population containing CD34⁺ cells may also contain a serum alternative (e.g., albumin, transferrin, knockout serum replacement (KSR), fatty acid, insulin, collagen precursor, trace elements, 2-mercaptoethanol, 3'-thiolglycerol, and ITS-supplement). The serum alternatives can be used singly, or in a combination of two or more.

Additionally, the medium for culturing the cell population containing CD34⁺ cells may contain one or more substances of the following: lipids, amino acids (e.g., non-essential amino acids), L-glutamine, vitamins, growth factors, cytokines, antibiotics, antioxidants, pyruvic acid, buffers, inorganic salts, etc.

The medium for culturing the cell population containing CD34⁺ cells is preferably a chemically-defined medium that does not contain materials with unknown components, such as serum, from the standpoint of reducing the lot-to-lot media variations and of preparing cell populations containing T cell progenitors with stable quality.

The medium for culturing the cell population containing CD34⁺ cells has a pH of typically 7.0 to 7.8, and preferably 7.2 to 7.6. The medium is preferably sterilized by a method such as filtration, UV irradiation, heat sterilization, or radiation irradiation before use in order to prevent contamination.

The medium for culturing the cell population containing CD34⁺ cells can be prepared in a solution form, a dry form, or a concentrated form (e.g., 2-fold to 1000-fold). The medium in a concentrated form can be diluted to an appropriate concentration for use. The liquid for use in diluting a medium of a dry form or a concentrated form is suitably selected from water, a buffered aqueous solution, a physiological saline solution, and the like.

The culture in the present invention may be either adhesion culture or suspension culture.

The production method of the present invention is preferably performed in the absence of feeder cells. Because the production method of the present invention enables culture in the absence of feeder cells, the production method enables stable production of cell populations containing T cell progenitors with uniform properties without contamination by materials with unknown components.

In the present specification, the phrase "in the absence of feeder cells" or "feeder-free" basically means not containing feeder cells, and not using a medium preconditioned by culturing feeder cells. Thus, the medium contains no substances such as growth factors and cytokines secreted from feeder cells.

The term "feeder cells" or "feeder" refers to cells that can be co-cultured with cells of different kind, and that provide an environment that supports and allows the cells to grow. Feeder cells may be of the same or different origin as that of the cells the feeder cells support. For example, feeders for human cells for use may be human dermal fibroblasts or human embryonic stem cells, or primary cultures of mouse embryo fibroblasts or immortalized mouse embryo fibroblasts. Feeder cells can be inactivated by exposure to irradiation or mitomycin C treatment.

The culture conditions for the production method of the present invention are not particularly limited. The culture temperature is, for example, about 37 to 42°C, and preferably about 37 to 39°C. The culture period is also not particularly limited and can be suitably determined by a person skilled in the art while monitoring, for example, the number of T cell progenitors. For example, the culture period can be 10 days or longer. The culture in the present invention may include as many times the passage as necessary to obtain a desired amount of the cell population containing T cell progenitors, or may include addition or replacement of the medium. The culture in the present invention can be performed by using a known CO₂ incubator.

The CD34⁺ cells for use in the production method of the present invention are CD34-positive (CD34⁺) cells. In an embodiment, the CD34⁺ cells are CD34-positive and CD7-negative (CD34⁺/CD7⁻) cells. In another embodiment, the CD34⁺ cells are hemogenic endothelial cells (HECs). The HECs are CD34-positive as well as CD45- and CD43-negative. The HECs can also be CD184- and CD73-negative. The HECs can be CD34⁺/CD43⁻/CD45⁻/CD184⁻/CD73⁻cells (because CD34⁺/CD43⁻/CD45⁻/CD184⁻/CD73⁻ cells do not express CD7, CD34⁺/CD43⁻/CD45⁻/CD184⁻/CD73⁻ cells are also referred to as CD34⁺/CD7⁻/CD43⁻/CD45⁻/CD184⁻/CD73⁻ cells). The HECs can also be CD14- and CD235a-negative. A cell population containing HECs can be isolated as CD14⁻/CD235a⁻/CD34⁺/CD43⁻/CD184⁻/CD73⁻. Thus, the use of HECs in step (1) enables the production of T cell progenitors with higher efficiency.

The production method of the present invention may further include step (A) of preparing iPS cells.

The method for preparing iPS cells can be the methods described above. The prepared iPS cells are induced to be differentiated into a cell population containing CD34⁺ cells, and the cell population containing CD34⁺ cells can be used in the culture of step (1). In the present invention, when pluripotent stem cells other than iPS cells are used, other pluripotent stem cells in place of iPS cells are prepared in step (A).

The pluripotent stem cells, cells for producing pluripotent stem cells, and cell population containing CD34⁺ cells for use in the present invention may be derived from humans or mammals other than humans (non-human mammals), and preferably humans. Examples of non-human mammals include mice, rats, hamsters, guinea pigs, rabbits, dogs, cats, pigs, cows, horses, sheep, and monkeys. It is also possible to use universalized iPS cells (i.e., cells with specific HLA types that do not cause immune rejection in many patients, or HLA knocked-out cells).

The production method of the present invention may further includes step (B) of inducing differentiation of pluripotent stem cells into a cell population containing CD34⁺ cells.

The differentiated cell population containing CD34⁺ cells can be used in culture of step (1). The differentiation of pluripotent stem cells into a cell population containing CD34⁺ cells can be induced according to a known method. If the pluripotent stem cells are iPS cells, a cell population containing CD34⁺ cells can be produced, for example, by inducing differentiation of hematopoietic progenitor cells according to the methods disclosed in WO2017/221975, WO2018/135646, or Cell Reports 2 (2012) 1722-1735.

The cell population containing CD34⁺ T cells used in the production method of the present invention may be a cell population containing CD34⁺ T cells differentiated from pluripotent stem cells (in particular, iPS cells (especially human iPS cells)). The percentage (number) of CD34⁺ T cells contained in the cell population containing CD34⁺ T cells is, for example, 5% or more, preferably 50% or more, and more preferably 90% or more. The upper limit is, for example, 100% or less.

The production method of the present invention may further includes step (C) of selecting a cell population containing hemogenic endothelial cells (CD34⁺/CD43⁻/CD45⁻/CD184⁻/CD73⁻ cells) from the cell population containing CD34⁺ cells obtained in step (B).

The isolation of a cell population containing hemogenic endothelial cells from the cell population containing CD34⁺ cells can be performed according to a known method. Examples of such methods include flow cytometry and magnetic cell separation. The use of the selected cell population containing hemogenic endothelial cells in the culture of step (1) enables the production of T cell progenitors with higher efficiency.

The cell population containing T cell progenitors produced by the production method of the present invention is a cell population derived from CD34⁺ T cells. The percentage (number) of T cell progenitors contained in the cell population containing T cell progenitors is, for example, 5% or more, preferably 15% or more, more preferably 25% or more, even more preferably 35% or more, and still even more preferably 45% or more. The upper limit is, for example, 100% or less.

The T cell progenitor inducer according to the present invention may contain the same ingredients as those of the production method of the present invention described above. When used in the production method described above, the T cell progenitor inducer according to the present invention can induce the differentiation into T cell progenitors.

The present invention further provides a medium for T cell progenitor differentiation and a kit for T cell progenitor differentiation. The medium and kit for T cell progenitor differentiation contain the same ingredients as those of the production method of the present invention described above. In an embodiment of the present invention, the kit for T cell progenitor differentiation contains an appropriate solid phase (e.g., a cell culture vessel material, and a cell culture carrier such as beads, membranes, glass slides) on which fibronectin or a fragment of fibronectin and/or DLL4 are immobilized. In an embodiment of the present invention, fibronectin or a fragment of fibronectin and/or DLL4 may be those immobilized on an appropriate solid phase beforehand, or those designed to be immobilized on an appropriate solid phase when used.

The T cell progenitors contained in the cell population obtained by the production method of the present invention may also be T cell progenitors into which a foreign gene has been introduced.

The "foreign gene" is a gene that is introduced from outside to allow T cell progenitors to express a desired protein, and can be selected appropriately depending on the intended use of the T cell progenitors.

The foreign gene can be a foreign gene encoding a receptor. For example, such foreign genes can be genes for expressing chimeric antigen receptors (CARs), T cell receptors (TCRs), synthetic T cell receptor and antigen receptors (STARs), or chimeric T cell antigen coupler (TAC) receptors. The foreign gene can be, for example, a gene for expressing a CAR and a gene for expressing a cytokine and/or chemokine. Similar to common or known CARs, the CAR expressed by the cells is basically composed of peptides at the following sites linked together via a spacer as necessary: (i) an antigen recognition site (e.g., a single-chain antibody, a ligand, or a peptide) that recognizes a cell surface antigen on a cancer cell; (ii) a transmembrane region; and (iii) a signal transduction region that induces T cell activation. Other foreign genes include suicide genes (e.g., iCas9 and HSV-TK) and cytokines (e.g., interleukins and chemokines).

The means for introducing a foreign gene into cells is not particularly limited, and various known or common means can be used. Typically, the foreign gene mentioned above is introduced into cells using an expression vector and then expressed. Expression vectors may be linear or circular, and may be non-viral vectors such as plasmids, viral vectors, or transposon-based vectors. The cells into which the foreign gene is introduced are not particularly limited, and may be at any stage of differentiation. Examples of such cells include pluripotent stem cells (in particular, iPS cells), CD34⁺ T cells, and T cell progenitors.

The technique for introducing an expression vector into cells can be any suitable technique depending on the embodiment. For example, an expression vector can be introduced into cells by known methods, such as viral infection, calcium phosphate method, lipofection, microinjection, and electroporation. Expression vectors can be prepared in a form suitable for use in each technique by known means or by using a commercially available kit (according to its instructions).

An expression vector can be introduced into cells by viral infection. Examples of viral vectors include retroviral vectors, lentiviral vectors, adenoviral vectors, and adeno-associated viral vectors. When using these viral vectors, a vector containing the desired foreign gene and the packaging vector (plasmid) of each virus may be transfected into host cells using the corresponding commercially available kit to produce recombinant viruses, and then the resulting recombinant viruses may be used to infect cells.

When there are multiple foreign genes, all of the foreign genes may be contained in a single expression vector, all of the foreign genes may be contained in separate expression vectors, or some of the multiple foreign genes may be contained in a single expression vector and the rest may be contained in separate expression vectors. When a single expression vector contains multiple foreign genes, the order in which the foreign genes are arranged from upstream to downstream is not particularly limited.

A foreign gene can be composed of a nucleic acid (polynucleotide) having a base sequence that encodes the desired protein or polypeptide depending on its amino acid sequence. A person skilled in the art would be able to design and create an expression vector capable of expressing the desired protein (polypeptide) in the cell. The nucleic acid contained in the expression vector may be prepared by a chemical DNA synthesis reaction or may be prepared (cloned) as cDNA.

In addition to the foreign gene, the expression vector may contain sequences, such as a promoter, terminator, enhancer, initiation codon, termination codon, polyadenylation signal, nuclear localization signal (NLS), and multiple cloning site (MCS), as necessary. The expression vector may further contain nucleic acids (base sequences) encoding "functional genes," such as reporter genes (e.g., genes encoding fluorescent proteins of various colors), drug selection genes (e.g., kanamycin resistance gene, ampicillin resistance gene, and puromycin resistance gene), and suicide genes (e.g., genes encoding diphtheria A toxin, herpes simplex virus thymidine kinase (HSV-TK), carboxypeptidase G2 (CPG2), carboxylesterase (CA), cytosine deaminase (CD), cytochrome P450 (cyt-450), deoxycytidine kinase (dCK), nitroreductase (NR), purine nucleoside phosphorylase (PNP), thymidine phosphorylase (TP), varicella-zoster virus thymidine kinase (VZV-TK), xanthine-guanine phosphoribosyltransferase (XGPRT), and inducible caspase 9).

The production method and T cell progenitor inducer of the present invention enable the production of T cell progenitors with high efficiency by using an aryl hydrocarbon receptor antagonist and an LSD1 degradation inducer in combination.

The cell population containing T cell progenitors produced by the production method of the present invention is useful in the treatment of animals (in particular, humans) in need of an increase of the number of T cells. The phase "in need of an increase of the number of T cells" refers to a condition in which the number of T cells in blood is reduced compared with that of healthy animals (e.g., a condition after treatment with an anticancer agent and/or radiotherapy, or a condition with acquired immunodeficiency syndrome (AIDS)). The T cell progenitors produced by the production method of the present invention are also useful in cell therapy, for example, for the recovery of T cells from thymic disorder after radiotherapy. T cell progenitors for use in cell therapy may be T cell progenitors transfected with a desired gene. Such cells can be used to treat genetic disorder (e.g., recombinase deficiency, recombinase regulation gene deficiency, or severe combined immunodeficiency (SCID)). Thus, the present invention provides a medicine containing T cell progenitors produced by the production method described above (which is sometimes referred to simply as "the medicine according to the present invention" in the present specification) (in particular, a cell-transplant therapeutic agent) and a cell therapy using the T cell progenitors. The T cell progenitors produced by the production method of the present invention may be used for autologous transplantation or allogeneic transplantation. Further, the T cell progenitors may be used in combination with other drugs. The T cell progenitors produced by the production method of the present invention have the property of differentiating into T cells after engrafting in the thymus, and then migrating into the blood, as shown in the Examples.

In such a cell therapy, from the standpoint of preventing rejection, the subject from which cells are isolated preferably has the same HLA type as a subject to which T cell progenitors are administered, and is more preferably the same as the subject to which T cell progenitors are administered.

The medicine according to the present invention can be, for example, a medicine (anticancer agent) for treating and preventing cancer that corresponds to a cell surface antigen (cancer-specific antigen) of a cancer cell that is targeted by the CAR expressed on the T cell progenitor. Examples of cancers that can be targeted by the medicine according to the present invention include cancers, such as adenocarcinoma, squamous cell carcinoma, adenosquamous carcinoma, undifferentiated carcinoma, large cell carcinoma, small cell carcinoma, skin cancer (e.g., melanoma and Merkel cell carcinoma), breast cancer, prostate cancer, bladder cancer, vaginal cancer, cervical cancer, head and neck cancer, uterine cancer, uterine cervical cancer, liver cancer, kidney cancer, pancreatic cancer, spleen cancer, lung cancer, non-small cell lung cancer, tracheal cancer, bronchial cancer, colon cancer, rectal cancer, small intestine cancer, large intestine cancer, gastric cancer, esophageal cancer, gallbladder cancer, testicular cancer, ovarian cancer, fallopian tube cancer, and nasopharyngeal cancer; cancer of bone tissue, cartilage tissue, adipose tissue, muscle tissue, vascular tissue, and hematopoietic tissue; sarcomas, such as chondrosarcoma, Ewing's sarcoma, rhabdomyosarcoma, malignant hemangioendothelioma, osteosarcoma, and soft tissue sarcomas; embryonal tumors, such as hepatoblastoma, medulloblastoma, nephroblastoma, neuroblastoma, pancreatoblastoma, pleuropulmonary blastoma, and retinoblastoma; germ cell tumor; lymphoma; leukemia, acute myeloid leukemia, and multiple myeloma. Preferred are cancer types susceptible to NK cells (e.g., melanoma, Merkel cell carcinoma, colon cancer, kidney cancer, breast cancer, ovarian cancer, fallopian tube cancer, uterine cervical cancer, liver cancer, lung cancer, non-small cell lung cancer, head and neck cancer, small intestine cancer, prostate cancer, bladder cancer, rectal cancer, pancreatic cancer, Ewing's sarcoma, rhabdomyosarcoma, nasopharyngeal cancer, esophageal cancer, biliary tract cancer, neuroblastoma, osteosarcoma, acute myeloid leukemia, multiple myeloma, lymphoma, and leukemia).

When the medicine according to the present invention is intended for cancer treatment, it can be used in combination with known anticancer agents. Examples of anticancer agents include alkylating agents, such as cyclophosphamide, bendamustine, ifosfamide, and dacarbazine; antimetabolites, such as pentostatin, fludarabine, cladribine, methotrexate, 5-fluorouracil, 6-mercaptopurine, and enocitabine; molecular-targeted drugs, such as rituximab, cetuximab, and trastuzumab; kinase inhibitors, such as imatinib, gefitinib, erlotinib, afatinib, dasatinib, sunitinib, and trametinib; proteasome inhibitors, such as bortezomib; calcineurin inhibitors, such as cyclosporine and tacrolimus; anticancer antibiotics, such as idarubicin, doxorubicin, and mitomycin C; plant alkaloids, such as irinotecan and etoposide; platinum-based drugs, such as cisplatin, oxaliplatin, and carboplatin; hormonal therapy drugs, such as tamoxifen and bicalutamide; and immunosuppressants, such as interferon, nivolumab, and pembrolizumab.

The medicine according to the present invention has low toxicity and can be administered safely. The medicine according to the present invention can be produced as an oral or parenteral formulation by, for example, mixing an effective amount of the cell population containing T cell progenitors with a pharmaceutically acceptable carrier according to known means. The medicine according to the present invention is usually produced as a parenteral formulation, such as injections, suspensions, and intravenous solutions. Parenteral administration methods include intravenous, intraarterial, intramuscular, intraperitoneal, and subcutaneous administration. Examples of pharmaceutically acceptable carriers include solvents, bases, diluents, excipients, analgesics, buffers, preservatives, stabilizers, suspending agents, isotonic agents, surfactants, and solubilization agents.

The dosage of the medicine according to the present invention can be appropriately determined according to various conditions such as the patient's weight, age, gender, and symptoms. For example, the medicine according to the present invention is typically administered in a single dose at a cell count of 1×10⁴ to 1x10¹⁰ cells, preferably 1×10⁵ to 1×10⁹ cells, and more preferably 5×10⁶ to 5×10⁸ cells. The medicine according to the present invention may be administered in a single dose or in multiple doses. The medicine according to the present invention can be made into a known form suitable for parenteral administration, for example, an injection or infusion. The medicine according to the present invention may also contain saline, phosphate buffered saline (PBS), medium, and the like to keep the cells stable. Examples of the medium include, but are not particularly limited to, RPMI, AIM-V, and X-VIVO10. The medicine may also contain pharmaceutically acceptable carriers (e.g., human serum albumin), preservatives, and the like for the purpose of stabilization. The medicine according to the present invention is applicable to mammals including humans.

As used in the present specification and claims, unless otherwise required by context, singular terms shall include pluralities and plural terms shall include the singular. Therefore, singular articles (e.g., "a," "an," and "the" in English) should be understood to also include the plural concept, unless otherwise specified.

### Examples

The present invention is described in more detail below with reference to Examples. However, the present invention is not limited to these Examples.

### Example 1: Derivation/Isolation of Cell Population Containing HECs

The cell population containing hemogenic endothelial cells (HECs) for use was a cell population obtained by differentiating iPS cells (Ff-I01s04: derived from peripheral blood mononuclear cells of a healthy individual, or Ff-WJc513: derived from umbilical cord blood mononuclear cells of a healthy individual) provided from the Center for iPS Cell Research and Application, Kyoto University, according to known methods (e.g., the methods disclosed in Cell Reports 2 (2012) 1722-1735, or WO2017/221975).

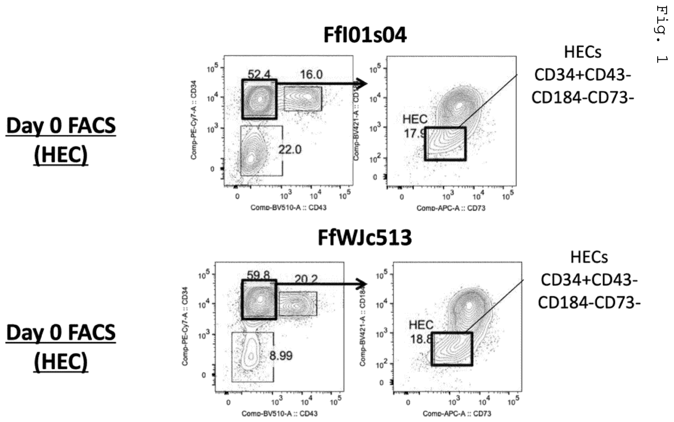

Specifically, Ff-I01s04 or Ff-WJc513 was seeded at 1×10⁶ cells/well in an ultra-low-attachment 6-well plate (Day 0). The cells were cultured under hypoxic conditions (5% 0₂) for 4 days in EB medium (StemPro-34 supplemented with 10 µg/ml human insulin, 5.5 µg/ml human transferrin, 5 ng/ml sodium selenite, 2 mM L-glutamine, 45 mM α-monothioglycerol, and 50 µg/ml ascorbic acid 2-phosphate) supplemented with 50 ng/ml BMP4, 50 ng/ml bFGF, 50 ng/ml VEGF, and 2 µM SB431542 (Day 4). Subsequently, culture was further performed in a medium supplemented with 50 ng/ml bFGF, 50 ng/ml VEGF, and 50 ng/ml SCF for 4 days (Day 8), thereby obtaining a floating cell population containing HECs. The floating cell population containing HECs were stained with the following antibody set (Table 1). As shown in Fig. 1, HECs were isolated as CD14⁻/CD235a⁻/CD34⁺/CD43⁻/CD184⁻/CD73⁻ using BD FACSAria^{™} Fusion.

**Table 1**

| | |
|---|---|
| Anti-CD34 antibody | Abcam PE/Cy7 |
| Anti-CD43 antibody | BD BV510 |
| Anti-CD73 antibody | BD APC |
| Anti-CD184 antibody | BD BV421 |
| Anti-CD235a antibody | BioLegend FITC |
| Anti-CD14 antibody | BioLegend APC/eF780 |

### Example 2: Differentiation from Cell Population Containing HECs into Cell Population Containing proT

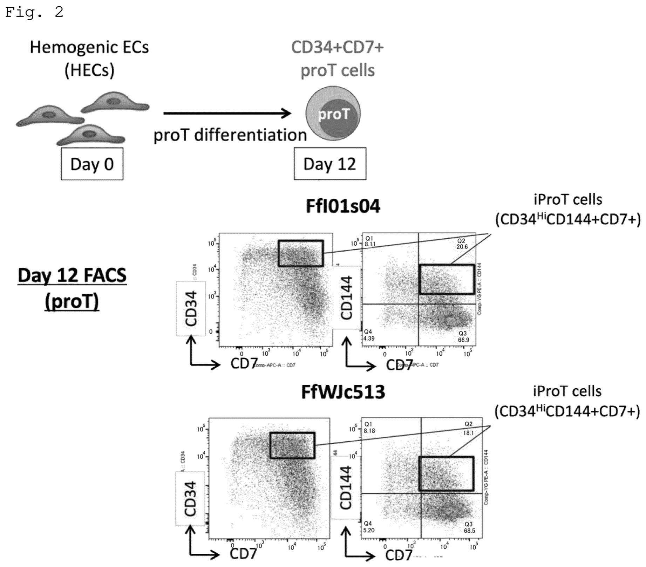

The cell population containing HECs isolated by the above method was seeded in an amount of 22,500 cells per well into a 6-well plate coated with RetroNectin (RN, Takara Bio Inc.) and Fc-tagged recombinant human delta-like 4 (DLL4-Fc, Sino Biological). The plate was prepared in a concentration of 5 µg/ml RN and 5 µg/ml DLL4-Fc. The basal medium used for the culture was ProT medium (IMDM-Glutamax supplemented with 0.5% recombinant human albumin (Recombumin Elite, Albumedix), 10 µg/ml human insulin, 5.5 µg/ml human transferrin, 6.7 ng/ml sodium selenite, 2 µg/ml ethanolamine, 55 µM 2-mercaptoethanol, 20 U/ml penicillin-streptomycin, 0.1% polyvinyl alcohol (Sigma-Aldrich), and 50 µg/ml ascorbic acid 2-phosphate) supplemented with 100 ng/ml recombinant human(rh) stem cell factor (SCF, R&D systems), 100 ng/ml rh Fms-like tyrosine kinase 3 ligand (Flt3L, PeproTech), 100 ng/ml rh thrombopoietin (TPO, PeproTech), 100 ng/ml rh interleukin 7 (IL-7, PeproTech), 10 ng/ml rh interleukin 6 (IL-6, PeproTech), 10 ng/ml rh interleukin 3 (IL-3, PeproTech), and 1 µM StemReginin-1 (SR1, Calbiochem), and the cells were cultured for 12 days. The resulting cells were collected by pipetting and immunostained with the following antibody set. Then, using a flow cytometer (FACSAria^{™} Fusion, BD Biosciences), it was confirmed that a cell population containing CD144⁺/CD34⁺/CD7⁺ T cell progenitors (proT) had been induced (Fig. 2).

**Table 2**

| | |
|---|---|
| Anti-CD144 antibody | BD PE |
| Anti-CD34 antibody | Abcam PE/Cy7 |
| Anti-CD45 antibody | BD BV510 |
| Anti-CD7 antibody | BioLegend APC |
| Anti-CD4 antibody | BD BV421 |
| Anti-CD8a antibody | BioLegend PerCP/Cy5.5 |

### Example 3: Promotion of proT Cell Differentiation by Co-addition of SR1 and UM171

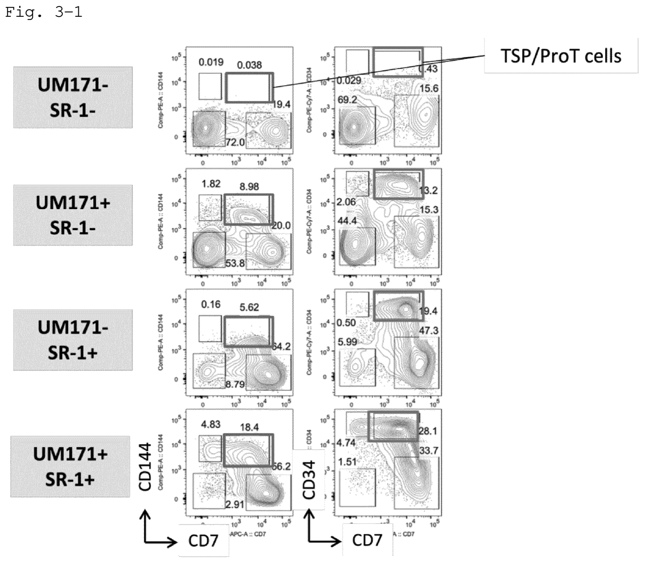

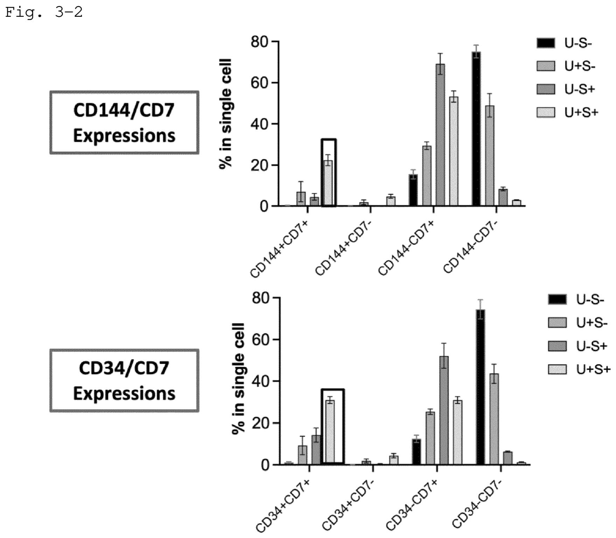

35 nM UM171 (Selleck Chemicals) was further added to the culture conditions of Example 2, and the cells were cultured for 12 days. The resulting cells were collected by pipetting and immunostained with the antibody set shown in Table 2. Then, the induction efficiency of CD144⁺/CD34⁺/CD7⁺ T cell progenitors (proT) was assessed with a flow cytometer (FACSAria^{™} Fusion, BD Biosciences). As a result, co-addition of SR1 and UM171 could induce a cell population containing a high proportion of proT cells (Fig. 3).

### Example 4: In Vivo Engraftment Assessment of Cell Population Containing proT Cells and Confirmation of T Cells in Blood

A cell population containing T cell progenitors induced from iPS cells in Example 2 or 3 was suspended at a dose of 1.5×10⁶ cells in 30 µl of an administration liquid (10 µl of D-PBS(-), 10 µl of 250 µg/ml anti-human-mouse IL-7 antibody (M25, Bio X Cell), and 10 µl of 50 µg/ml rhIL-7), and transplanted into the liver of two-day-old neonate NOD.Cg-Prkdc^{scid}Il2rg^{tm1Wjl}/SzJ (NSG) mice (The Jackson Laboratory Japan, Inc.). The thymus was collected 4 weeks after administration, and the blood was collected 8 and 12 weeks after administration, after which whether human CD45-positive T cells were present was assessed.

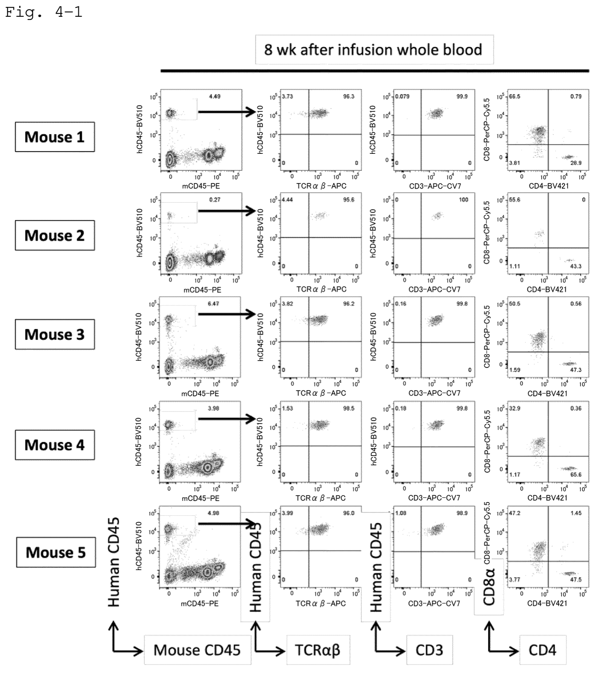

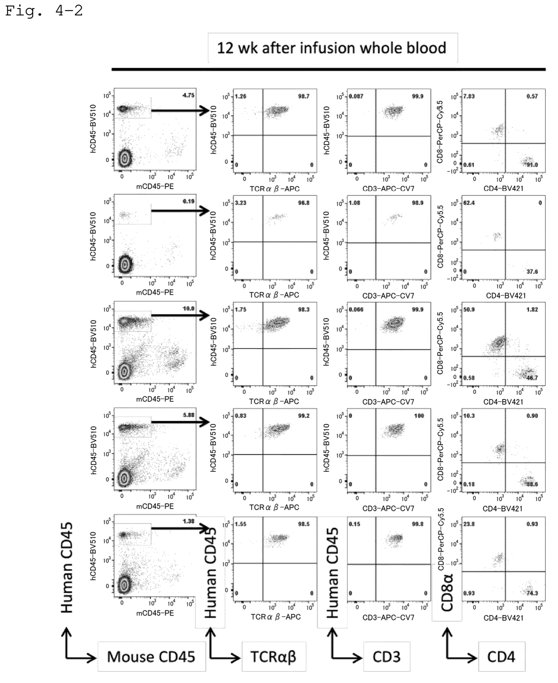

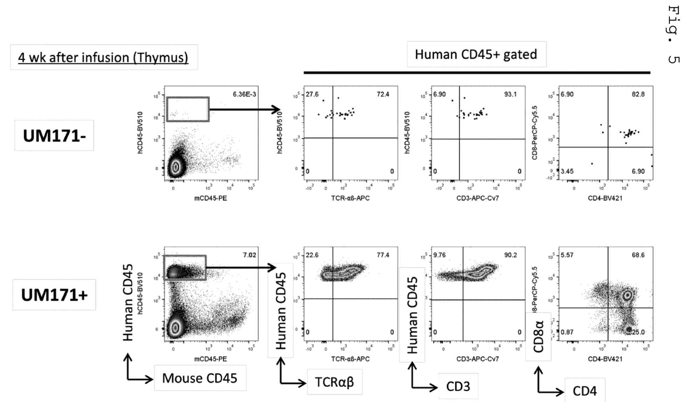

In the group administered with the cell population, CD4⁺CD8⁺-double-positive cells of human CD45⁺/CD3⁺/aPTCR⁺/CD4⁺/CD8a⁺ were confirmed in the thymus. Furthermore, in the blood samples collected 8 and 12 weeks after transplantation using the cells induced by the method of Example 3, the presence of CD4⁺-single-positive cells of CD45⁺/CD3⁺/aPTCR⁺/CD4⁺/CD8a⁻ and CD8⁺-single-positive cells of CD45⁺/CD3⁺/aPTCR⁺/CD4⁻/CD8a⁺ was confirmed (Fig. 4). When the cells induced by the methods of Examples 2 and 3 were compared and assessed, the cells induced by co-addition with UM171 showed higher thymic engraftment ability (Fig. 5).

### Example 5: CAR-proT Cell Production and In Vivo Anticancer Activity

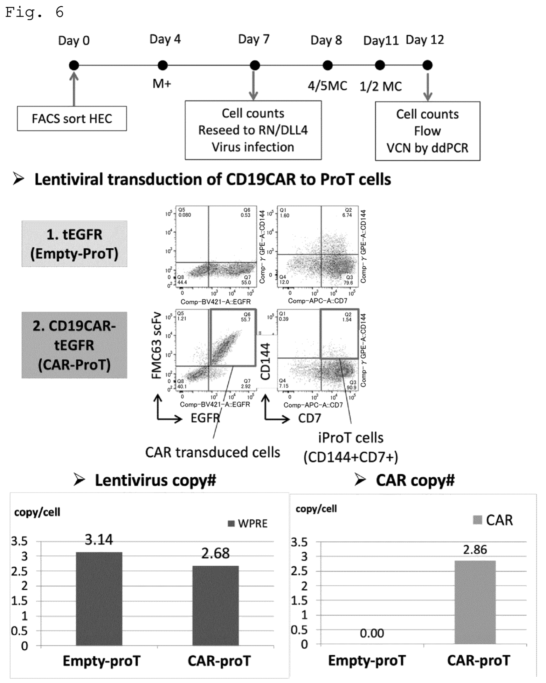

To assess chimeric antigen receptor (CAR)-specific antitumor effects in vivo, during the process of inducing differentiation of a cell population containing proT cells from a cell population containing HECs, a CAR against CD19, which is a B cell tumor antigen, was transduced into the differentiated cells using a lentiviral vector. 7 days after the start of differentiation induction, the lentiviral vector was added to the culture. 4 days after gene transfer (12 days after the start of differentiation induction), the cultured cells were collected and immunostained with the antibody set shown in Table 3, followed by expression analysis using a flow cytometer. As a result, it was confirmed that CAR-expressing proT cells could be produced (Fig. 6).

**Table 3**

| | |
|---|---|
| Anti-FMC63scFv antibody | AcroBio FITC |
| Anti-CD144 antibody | BD PE |
| Anti-CD34 antibody | Abcam PE/Cy7 |
| Anti-CD45 antibody | BD BV510 |
| Anti-CD7 antibody | BioLegend APC |
| Anti-CD4 antibody | BD APC/H7 |
| Anti-CD8a antibody | BioLegend PerCP/Cy5.5 |
| Anti-EGFR antibody | BioLegend BV421 |

A cell population containing CAR-expressing proT cells produced as described above was suspended at a dose of 1.5×10⁶ cells in 30 µl of an administration liquid (10 µl D-PBS(-), 10 µl of 250 µg/ml anti-human-mouse IL-7 antibody (M25, Bio X Cell), and 10 µl of 50 µg/ml rhIL-7), and transplanted into the liver of two-day-old neonate NOD.Cg-Prkdc^{scid}Il2rg^{tm1Wjl}/SzJ (NSG) mice (The Jackson Laboratory Japan, Inc.). The blood was collected weekly starting 7 weeks after administration, and stained with the antibody set shown in Table 3, followed by expression analysis using a flow cytometer to assess the presence of human CD45-positive CAR-T cells. Additionally, 8 weeks after administration, 1×10⁵ cells of CD19-expressing B cell tumor line NALM-6 were intravenously administered per mouse, and antitumor effects were assessed by monitoring luciferase luminescence.

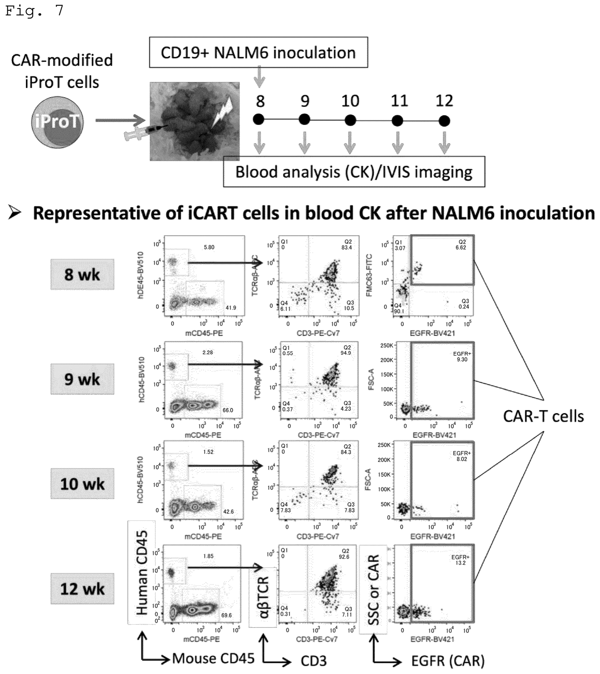

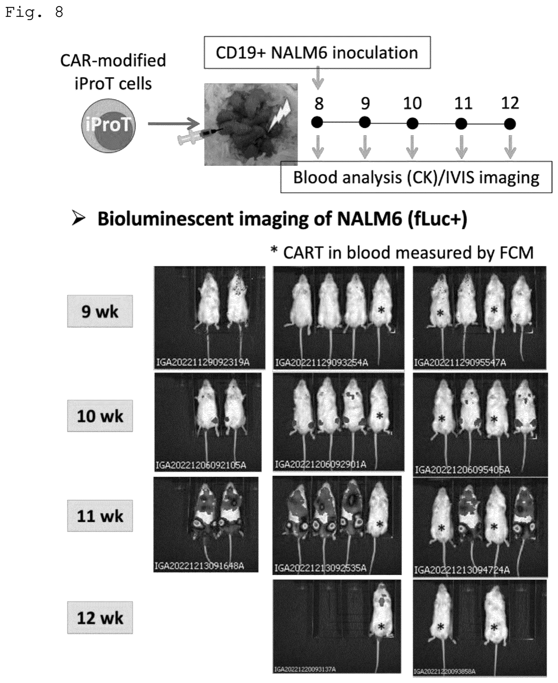

As a result, the presence of CD19CAR⁺/CD45⁺/CD3⁺/αβTCR⁺ cells in the blood was confirmed in the CAR-expressing proT cell transplant group (Fig. 7). Furthermore, in individuals in which CAR-T cells were detected in the blood, luminescence derived from NALM-6 was not detected, and tumor growth was suppressed in these individuals (Fig. 8).

The present application is based on Japanese Patent Application No. 2023-91294, filed on June 2, 2023, in Japan, the disclosure of which is incorporated herein by reference in its entirety.

## Claims

1. A method for producing a cell population containing a T cell progenitor, the method comprising the following step:
(1) culturing a cell population containing a CD34⁺ cell in the presence of an aryl hydrocarbon receptor antagonist and an LSD1 degradation inducer.

2. The method according to claim 1, wherein the aryl hydrocarbon receptor antagonist is Stemregenin-1 (SR1).

3. The method according to claim 1, wherein the LSD1 degradation inducer is UM171.

4. The method according to claim 1, wherein step (1) is a step of culturing a CD34⁺ cell in the further presence of fibronectin or a fragment of fibronectin, and/or Delta-like 4 (DLL4).

5. The method according to claim 4, wherein step (1) is a step of culturing a CD34⁺ cell in the further presence of at least one member selected from the group consisting of a stem cell factor (SCF), interleukin-3 (IL-3), and interleukin-6 (IL-6).

6. The method according to claim 1, wherein the CD34⁺ cell is a CD34⁺/CD43⁻/CD184⁻/CD73⁻ cell.

7. The method according to claim 1, wherein the cell population containing a CD34⁺ cell is derived from an induced pluripotent stem cell.

8. The method according to claim 1, wherein the T cell progenitor comprises a foreign gene encoding a receptor.

9. A cell population containing a T cell progenitor obtained by the method according to claim 1.

10. A medicine comprising the cell population containing a T cell progenitor of claim 9.

11. The medicine according to claim 10, which is a preventive and/or therapeutic agent for cancer.

12. A T cell progenitor inducer comprising an aryl hydrocarbon receptor antagonist and an LSD1 degradation inducer.

13. The inducer according to claim 12, wherein the aryl hydrocarbon receptor antagonist is SR1.

14. The inducer according to claim 12, wherein the LSD1 degradation inducer is UM171.

15. The inducer according to claim 12, further comprising fibronectin or a fragment of fibronectin, and/or DLL4.

16. The inducer according to claim 15, further comprising at least one member selected from the group consisting of SCF, IL-3, and IL-6.

17. A method for preventing and/or treating cancer, comprising administering the cell population containing a T cell progenitor of claim 9 to a subject in need thereof.

18. The cell population containing a T cell progenitor of claim 9, for use in the prevention and/or treatment of cancer.

19. Use of the cell population containing a T cell progenitor of claim 9 in the production of a medicine for preventing and/or treating cancer.
